# EUROPEAN PATENT APPLICATION

(11) **EP 4 703 375 A1**
(43) Date of publication of application: **04.03.2026**
(21) Application number: 24796081.8
(22) Date of filing: 24.04.2024
(51) Int. Cl.: C07K 14/725, C07K 14/705, C07K 16/28, C12M 3/02, C12N 5/0783

(54) **T CELL RECEPTOR AND USE THEREOF**

(30) Priority: 25.04.2023 WO PCT/CN2023/090456
(71) Applicant: Beijing Grit Biotherapeutics Co., Ltd., Shanghai 200120 (CN); Shanghai Grit Biotechnology Co., Ltd., Shanghai 200120 (CN)
(72) Inventor: LIU, Yarong, Shanghai 200120 (CN); JIA, Luying, Shanghai 200120 (CN); CUI, Jun, Shanghai 200120 (CN); YUAN, Hui, Shanghai 200120 (CN); LIU, Zhenjiang, Shanghai 200120 (CN); LIU, Yanbin, Shanghai 200120 (CN)
(74) Representative: Fish & Richardson P.C.
(86) International application number: PCT/CN2024/089425
(87) International publication number: WO 2024/222701

(57) **Abstract**

The present invention relates to the field of biomedicines and particularly provides a T cell receptor (TCR) and use thereof. The TCR can be used for modifying cells in order to improve or promote the expression of the proliferation capability, killing capability, and/or directed differentiation capability of the TCR cell.

## Description

### TECHNICAL FIELD

The present invention relates to the field of biomedicine, and in particular to a T cell receptor and uses thereof.

### BACKGROUND

Natural killer cells (NKT) are a special type of T lymphocyte subset with the dual properties of T cells and NK cells. NKT cells can express two receptors, TCR of T cells and NKR-P1 of NK cells. Under the mediation of TCR and NKR, NKT cells can produce a large number of cytokines. Among them, the T cell receptor (TCR) mediates the recognition of peptide epitopes bound to major histocompatibility complex (MHC) molecules to exert adaptive cellular immunity. Normally, the half-life of infused NKT cells in the patient's body is about 2 weeks. The effective period is short, and repeated infusions are required. In addition, NKT cells themselves lack specific antibodies and are insufficiently enriched around tumors or in tumor nests, which restricts the targeted effect treatment of NKT cells. In another aspect, NKT from peripheral blood is relatively rare, accounting for only 0.1-0.5%, and is not easy to use for cell therapy.

Therefore, there is an urgent need in the art for a class of TCR molecules, which, after being transduced into stem cells (including HSC, iPSC, ESC), can enable the stem cells to better differentiate in the direction of NKT, thereby obtaining a large number of NKT cells that can be used for cell therapy.

### SUMMARY

The present invention provides a T cell receptor (TCR) and its uses, which can have one or more of the following advantages: the ability to be expressed in cells, the ability to increase the proliferation of cells expressing the TCR, the ability to increase the killing ability of cells expressing the TCR, and/or the ability to promote the directed differentiation of cells expressing the TCR.

In one aspect, the present invention provides a T cell receptor (TCR) or an antigen-binding fragment thereof, wherein the TCR comprises a β chain variable region (Vβ), the Vβ comprises a CDR3, and the CDR3 of the Vβ comprises the CDR3 sequence of the Vβ provided by the present invention.

In another aspect, the present invention provides a fusion protein comprising the TCR or antigen-binding fragment thereof described in the present invention.

In another aspect, the present invention provides a nucleic acid molecule encoding the TCR or antigen-binding fragment thereof described in the present invention and/or the fusion protein described in the present invention.

In another aspect, the present invention provides a vector comprising the nucleic acid molecule of the present invention.

In another aspect, the present invention provides a composition comprising the TCR or antigen-binding fragment thereof described in the present invention, the fusion protein described in the present invention, the nucleic acid molecule described in the present invention, and/or the vector described in the present invention, and a pharmaceutically acceptable adjuvant.

In another aspect, the present invention provides a cell comprising the TCR or antigen-binding fragment thereof described in the present invention, the fusion protein described in the present invention, the nucleic acid molecule described in the present invention, the vector described in the present invention and/or the composition described in the present invention.

In another aspect, the present invention provides a kit comprising the TCR or antigen-binding fragment thereof described in the present invention, the fusion protein described in the present invention, the nucleic acid molecule described in the present invention, the vector described in the present invention, the composition described in the present invention and/or the cell described in the present invention.

In another aspect, the present invention provides a method for influencing cell growth, comprising administering the TCR or antigen-binding fragment thereof described in the present invention, the fusion protein described in the present invention, the nucleic acid molecule described in the present invention, the vector described in the present invention, the composition described in the present invention, the cell described in the present invention and/or the kit described in the present invention.

In another aspect, the present invention provides the TCR or antigen-binding fragment thereof of the present invention, the fusion protein of the present invention, the nucleic acid molecule of the present invention, the vector of the present invention, the composition of the present invention, the cell of the present invention and/or the kit of the present invention for use in the preparation of a medicament, where in the medicament is for preventing and/or treating a disease and/or symptom.

In another aspect, the present invention provides the TCR or antigen-binding fragment described in the present invention, the fusion protein described in the present invention, the nucleic acid molecule described in the present invention, the vector described in the present invention, the composition described in the present invention, the cell described in the present invention and/or the kit described in the present invention, which are used to prevent and/or treat a disease and/or symptom.

In another aspect, the present invention provides a method for preventing and/or treating a disease and/or symptom, comprising administering the TCR or antigen-binding fragment thereof described in the present invention, the fusion protein described in the present invention, the nucleic acid molecule described in the present invention, the vector described in the present invention, the composition described in the present invention, the cell described in the present invention and/or the kit described in the present invention.

Those skilled in the art can easily discern other aspects and advantages of the present invention from the detailed description below. In the detailed description below, only exemplary embodiments of the present invention are shown and described. As will be appreciated by those skilled in the art, the content of the present invention enables those skilled in the art to make changes to the disclosed specific embodiments without departing from the spirit and scope of the invention to which the present invention relates. Accordingly, the drawings and description of the present invention are merely exemplary and not restrictive.

### BRIEF DESCRIPTION OF THE DRAWINGS

The features and advantages of the invention can be better understood by referring to the exemplary embodiments and drawings described in detail below. The drawings are briefly described as follows:
FIG. 1 shows the results of sorting CD45-positive TCRVα24-positive cell populations.
FIG. 2 shows the single-cell transcriptome sequencing results of cells corresponding to each group of TCR (001-007).
FIG. 3 shows the results of tetramer-positive cell population sorting.
FIG. 4 shows the single-cell transcriptome sequencing results of cells corresponding to each group of TCR (008-015).
FIG. 5 shows the detection results of TCR expression in each group.
FIGs. 6A-6B show the cell phenotype results in cells expressing the TCR of the present invention.
FIG. 7 shows the target cell killing effect of cells expressing the TCR of the present invention.
FIG. 8 shows the target cell killing effect of peripheral blood-derived cells expressing the TCR of the present invention.

### DETAILED DESCRIPTION

The following is an explanation of the implementation of the present invention by means of specific embodiments. Those skilled in the art can easily understand other advantages and effects of the present invention from the contents disclosed in this specification.

### Definition of terms

In the present invention, the term "T cell receptor" or "TCR" generally refers to an endogenous or engineered T cell receptor. For example, a TCR may include an extracellular antigen binding domain that binds to a specific antigen epitope in an MHC molecule. A TCR may include a TCRα polypeptide chain and a TCRβ polypeptide chain. A "tumor-specific TCR" refers to a TCR that specifically recognizes a tumor antigen expressed by a tumor cell. A "TCR cell" refers to a cell expressing a recombinant TCR. The "antigen binding domain" of a TCR refers to a polypeptide that the TCR specifically binds to an antigen with a higher degree of specificity due to its primary, secondary or tertiary sequence and/or post-translational modification and/or charge. The antigen binding domain may be derived from any part or fragment of a TCR that, as a part of a TCR, retains the antigen binding activity of the TCR. The antigen binding portion encompasses the portion that has the ability to detect, treat or prevent cancer. The TCR antigen binding domain may comprise about 10-95% or more, such as about 10%, about 25%, about 30%, about 50%, about 68%, about 80%, about 90%, about 95% or more of the full-length TCR. The antigen binding domain may comprise an antigen recognition portion of any one or both of the α chain and β chain of the TCR, such as one or more of the complementary determining regions CDR1, CDR2 and CDR3 of the variable region of the α chain and/or β chain of the TCR. The antigen binding domain may comprise the following amino acid sequence: CDR1 of the α chain, CDR2 of the α chain, CDR3 of the α chain, CDR1 of the β chain, CDR2 of the β chain, CDR3 of the β chain, or any combination thereof. Preferably, the antigen binding domain comprises the amino acid sequence of CDR1, CDR2 and CDR3 of the α chain of TCR or the amino acid sequence of CDR1, CDR2 and CDR3 of the β chain; or the amino acid sequence of CDR1, CDR2, CDR3 of all α chains and β chains. In one embodiment of the present invention, the antigen binding portion may include, for example, a variable region of TCR, which includes a combination of the above-mentioned CDR regions. In this aspect, the antigen binding portion may comprise the amino acid sequence of the variable region of the α chain of TCR (Vα), the amino acid sequence of the variable region of the β chain (Vβ), or the amino acid sequence of both Vα and Vβ. In one embodiment of the present invention, the antigen binding portion may comprise a combination of a variable region and a constant region. In this aspect, the antigen binding portion may comprise the full length α or β chain, or both α and β chains of a TCR.

For example, the α and β chains of a TCR are generally considered to each have two "domains" or "regions" called variable and constant domains/regions. The terms "domain" and "region" are used interchangeably herein. The variable domain consists of a concatenation of variable regions and linker regions. In the description and claims of the present invention, the term "TCRα variable domain" therefore refers to the concatenation of the TRAV and TRAJ regions, and the term TCRα constant domain refers to the extracellular TRAC region or to a C-terminal truncated TRAC sequence. Similarly, the term "TCRβ variable domain" refers to the concatenation of the TRBV and TRBD/TRBJ regions, and the term TCRβ constant domain refers to the extracellular TRBC region or to a C-terminal truncated TRBC sequence. Similarly, the linker region of a TCR is defined by the unique IMGT TRAJ and TRBJ nomenclature, and the constant region is defined by the IMGT TRAC and TRBC nomenclature. The β chain variable region is referred to by the abbreviation TRBD in the IMGT nomenclature, and the concatenated TRBD/TRBJ regions are generally considered together as the linker region.

The example of TCR includes but is not limited to full-length TCR, the antigen binding fragment of TCR, the soluble TCR lacking transmembrane region and cytoplasmic region, the single-chain TCR comprising the variable region attached by flexible linker of TCR, the TCR chain connected by modified disulfide bond, monospecific TCR, multispecific TCR (including bispecific TCR), TCR fusion, human TCR, humanized TCR, chimeric TCR, recombinant TCR and synthetic TCR. The term covers wild-type TCR and genetically modified TCR (e.g., chimeric TCR comprising chimeric TCR chain, the chimeric TCR chain comprising the first part of TCR from the first species and the second part of TCR from the second species). In certain embodiments, TCR includes transmembrane region. In certain embodiments, TCR includes costimulatory signaling region.

In the present invention, the term "vector" generally refers to a vector by which a polynucleotide sequence (e.g., a foreign gene) can be introduced into a host cell in order to obtain the desired gene expression of the introduced nucleotide sequence. Cloning vectors may include, for example, plasmids, bacteriophages, viruses, etc. Another type of vector is a viral vector that connects the nucleic acid construct to be transported to the viral genome. Viral vectors are capable of autonomously replicating in the host cells into which they are introduced, or of integrating themselves into the host cell genome, thereby replicating together with the host genome. In addition, certain vectors are capable of directing the expression of genes operably linked thereto. Such vectors are referred to herein as "recombinant expression vectors" or simply "expression vectors". In some embodiments, the vector is a viral vector (e.g., a replication-defective retrovirus, adenovirus, and adeno-associated virus).

In the present invention, the term "cancer" or "cancer cell" generally refers to a cell that divides in an uncontrolled manner. Examples of such cells include cells with an abnormal state or condition characterized by rapidly proliferating cell growth. The term is intended to include cancerous growths (e.g., tumors), carcinogenic processes, metastatic tissues, and malignantly transformed cells, tissues, or organs, regardless of the histopathological type or infiltration stage. Cancer cells can form solid tumors or excessive tumor cells (e.g., blood cancers) in the blood. Alternatively or additionally, it can include all types of cancerous growths or carcinogenic processes, metastatic tissues, or malignantly transformed cells, tissues, or organs, regardless of the histopathological type or infiltration stage.

In the present invention, the term "pharmaceutically acceptable" or "pharmacologically compatible" generally refers to a material that does not have undesirable biological or other properties. For example, the material can be incorporated into a pharmaceutical composition administered to an individual without causing any significant undesirable biological effects or interacting in a harmful manner with any other component of the composition containing it. Pharmaceutically acceptable adjuvants or excipients preferably meet the required toxicological testing standards and manufacturing testing standards, and/or are included in the inactive ingredient guide established by the Food and Drug Administration.

In the present invention, the term "immune cell" generally refers to a cell that participates in an immune response, such as a cell that promotes an immune effector response. Examples of immune cells include, but are not limited to, T cells, B cells, natural killer (NK) cells, NKT cells, mast cells, granulocytes, monocytes, lymphocytes, and macrophages. For example, the immune cells of the present invention may include cells derived from artificial pluripotent stem (iPS) cells, PBMC cells, and/or tumor infiltrating lymphocytes. For example, the immune cells of the present invention may be obtained by differentiation of iPS cells. The term also includes engineered immune cells, such as immune cells that are genetically modified by adding exogenous genetic material in the form of DNA or RNA to the total genetic material of the cell.

In the present invention, the term "PBMC" or "human peripheral blood mononuclear cell" generally refers to a cell with a single nucleus in peripheral blood. For example, any blood cell (i.e., lymphocyte, monocyte or macrophage) with a round nucleus. These blood cells are key components of the immune system to resist infection and adapt to invaders. Lymphocyte populations are composed of CD4 ⁺ and CD8 ⁺ T cells, B cells, NKT cells and natural killer cells, CD14 ⁺ monocytes and basophils/neutrophils/eosinophils/dendritic cells. Typically, these cells are separated from whole blood using FICOLL ^{™} (a hydrophilic polysaccharide that stratifies blood), wherein monocytes and lymphocytes form a buffy coat under the plasma layer. For example, "PBMC" refers to a cell population comprising at least T cells, and optionally NK cells, NKT cells and antigen presenting cells.

In the present invention, the term "killing ability" refers to the killing of cells by contacting the cells with an effective amount of active substances. For example, the TCR of the present invention can enhance the killing ability of cells. For example, cells expressing the TCR of the present invention can show enhanced killing ability. For example, the combination of cells expressing the TCR of the present invention and other immune cells, antibodies, immunoconjugates (e.g., conjugates of binding molecules targeting immune checkpoints and other active molecules), and/or bispecific/multispecific molecules can show enhanced killing ability. The method may include killing cells that express positive antigens, optionally in the presence of effector cells, such as by CDC, apoptosis, ADCC, phagocytosis, or by a combination of two or more of these mechanisms.

In the present invention, the terms "about" and "approximately" generally refer to a statistically significant numerical range. Such a range can be within an order of magnitude of a given value or range, can be included within 50%, within 20%, within 10%, or within 5%. The permissible variation contained in the term "about" or "approximately" can depend on the specific system under study, and can be easily understood by those of ordinary skill in the art. The terms "above", "below", "at most" and "at least" can include this number.

### Embodiments of the Invention

In one aspect, the present invention provides a T cell receptor (TCR) or an antigen binding fragment thereof, wherein the TCR comprises a β chain variable region (Vβ), the Vβ comprises a CDR3, and the CDR3 of the Vβ comprises a sequence shown in any one of SEQ ID NOs: 14-26, or an amino acid sequence having at least 80% to 99%, such as at least 80%, 85%, 90%, 91%, 92%, 93%, 94%, 95%, 96%, 97%, 98% or 99% sequence identity thereto.

For example, the TCR comprises a β chain variable region (Vβ), the Vβ comprises CDR1 and CDR2, and the CDR1 of the Vβ comprises the sequence shown in SEQ ID NO: 28, or an amino acid sequence having at least 80% to 99%, such as at least 80%, 85%, 90%, 91%, 92%, 93%, 94%, 95%, 96%, 97%, 98% or 99% sequence identity thereto. For example, the TCR comprises a β chain variable region (Vβ), the Vβ comprises CDR1 and CDR2, and the CDR2 of the Vβ comprises the sequence shown in SEQ ID NO: 27, or an amino acid sequence having at least 80% to 99%, such as at least 80%, 85%, 90%, 91%, 92%, 93%, 94%, 95%, 96%, 97%, 98% or 99% sequence identity thereto.

For example, the TCR comprises a β chain variable region (Vβ), wherein the Vβ comprises the sequence shown in any one of SEQ ID NOs: 1-13, or an amino acid sequence having at least 80% to 99%, such as at least 80%, 85%, 90%, 91%, 92%, 93%, 94%, 95%, 96%, 97%, 98% or 99% sequence identity thereto.

For example, the TCR comprises a β chain constant region, which comprises the sequence shown in SEQ ID NO: 29 or 30, or an amino acid sequence having at least 80% to 99%, such as at least 80%, 85%, 90%, 91%, 92%, 93%, 94%, 95%, 96%, 97%, 98% or 99% sequence identity thereto.

For example, the TCR comprises an α chain variable region (Vα), the Vα comprises CDR3, and the CDR3 of the Vα comprises the sequence shown in SEQ ID NO: 31, or an amino acid sequence having at least 80% to 99%, such as at least 80%, 85%, 90%, 91%, 92%, 93%, 94%, 95%, 96%, 97%, 98% or 99% sequence identity thereto.

For example, the TCR comprises an α chain variable region (Vα), wherein Vα comprises CDR1 and CDR2, and the CDR1 of the Vα comprises the sequence shown in SEQ ID NO: 33, or an amino acid sequence having at least 80% to 99%, such as at least 80%, 85%, 90%, 91%, 92%, 93%, 94%, 95%, 96%, 97%, 98% or 99% sequence identity thereto. For example, the TCR comprises an α chain variable region (Vα), the Vα comprises CDR1 and CDR2, and the CDR2 of the Vα comprises the sequence shown in SEQ ID NO: 32, or an amino acid sequence having at least 80% to 99%, such as at least 80%, 85%, 90%, 91%, 92%, 93%, 94%, 95%, 96%, 97%, 98% or 99% sequence identity thereto.

For example, the TCR comprises an α chain variable region (Vα), wherein Vα comprises the sequence shown in SEQ ID NO: 34, or an amino acid sequence having at least 80% to 99%, such as at least 80%, 85%, 90%, 91%, 92%, 93%, 94%, 95%, 96%, 97%, 98% or 99% sequence identity thereto.

For example, the TCR comprises an α chain constant region, which comprises the sequence shown in SEQ ID NO: 35, or an amino acid sequence having at least 80% to 99%, such as at least 80%, 85%, 90%, 91%, 92%, 93%, 94%, 95%, 96%, 97%, 98% or 99% sequence identity thereto.

For example, the TCR comprises an α chain, the full length of which comprises the sequence shown in SEQ ID NO: 36, or an amino acid sequence having at least 80% to 99%, such as at least 80%, 85%, 90%, 91%, 92%, 93%, 94%, 95%, 96%, 97%, 98% or 99% sequence identity thereto.

For example, the sequence mentioned in the present invention comprises a sequence that is at least about 95% homologous to the sequence. For example, the sequence mentioned in the present invention comprises a sequence that is at least 80% to 99% homologous to the sequence, such as at least about 80%, at least about 85%, at least about 90%, at least about 95%, at least about 96% of the sequence, at least about 97% of the sequence, at least about 98% of the sequence, at least about 99% homologous to the sequence. Preferably, the sequence mentioned in the present invention also comprises a sequence after one or more amino acid insertions, deletions or substitutions, especially conservative amino acid substitutions.

For example, the TCR comprises an α chain variable domain and a β chain variable domain (SEQ ID NO number) of a TCR molecule selected from the following table:

| TCR No. | β chain variable region | β chain CDR1 | β chain CDR2 | β chain CDR3 | α chain variable region | α chain CDR1 | α chain CDR2 | α chain CDR3 |
|---|---|---|---|---|---|---|---|---|
| TCR-001 | 1 | 28 | 27 | 14 | 34 | 33 | 32 | 31 |
| TCR-002 | 2 | | | 15 | | | | |
| TCR-003 | 3 | | | 16 | | | | |
| TCR-004 | 4 | | | 17 | | | | |
| TCR-005 | 5 | | | 18 | | | | |
| TCR-008 | 6 | | | 19 | | | | |
| TCR-009 | 7 | | | 20 | | | | |
| TCR-010 | 8 | | | 21 | | | | |
| TCR-011 | 9 | | | 22 | | | | |
| TCR-012 | 10 | | | 23 | | | | |
| TCR-013 | 11 | | | 24 | | | | |
| TCR-014 | 12 | | | 25 | | | | |
| TCR-015 | 13 | | | 26 | | | | |
| TCR-006 | 39 | NT | NT | NT | 41 | NT | NT | NT |
| TCR-007 | 40 | NT | NT | NT | 34 | 33 | 32 | 31 |

In certain embodiments, the present invention provides one, two or all three of the CDRs of Vα or Vβ provided herein with the TCR of the present invention. For example, the CDRs can be defined according to the Kabat or IMGT numbering system and division method.

In one aspect, the present invention provides the use of the TCR of the present invention in preparing a fusion protein. For example, the TCR of the present invention is operably linked to other molecules. In one aspect, the present invention provides a fusion protein comprising the TCR of the present invention or an antigen-binding fragment thereof.

The present invention also provides TCR-derived antibodies or derived CARs. The present disclosure also provides antibodies or antigen-binding fragments thereof or derived CARs containing any one or more CDRs as described in the present invention. In some embodiments, the derived antibody or antigen-binding fragment or derived CAR contains variable heavy and light chains, and the variable heavy and light chains are included in the α chain CDR1, CDR2 and/or CDR3 and CDR1, CDR2 and/or CDR3 contained in the β chain. In some embodiments, the antibody or antigen-binding fragment comprises one or more CDRs with at least about 80% to 99% identity to the CDR sequence of the present invention, or one or more CDRs with about 80%, 85%, 90%, 91%, 92%, 93%, 94%, 95%, 96%, 97%, 98% or 99% sequence identity thereto.

In one aspect, the present invention provides a nucleic acid, wherein the nucleic acid molecule encodes the TCR or antigen-binding fragment thereof described in the present invention and/or the fusion protein described in the present invention.

In another aspect, the present invention provides a vector, which may comprise the nucleic acid of the present invention.

In another aspect, the present invention provides a composition comprising the TCR or antigen-binding fragment thereof of the present invention, the fusion protein of the present invention, the nucleic acid molecule of the present invention, and/or the vector of the present invention, and optionally a pharmaceutically acceptable adjuvant. For example, excipients such as disintegrants, binders, lubricants, diluents, buffers, isotonicities, preservatives, wetting agents, emulsifiers, dispersants, stabilizers, and cosolvents.

In another aspect, the present invention provides a cell, which may contain the TCR or its antigen-binding fragment described in the present invention, the fusion protein described in the present invention, the nucleic acid molecule described in the present invention, the vector described in the present invention and/or the composition described in the present invention.

For example, the cell may comprise an immune cell. For example, the cell may comprise NK cells, NKT cells and/or T cells. For example, the cell may comprise cells derived from artificial pluripotent stem (iPS) cells, totipotent stem cells, pluripotent stem cells, PBMC cells and/or tumor infiltrating lymphocytes. For example, the cell of the present invention may be obtained by differentiation of iPS cells. For example, the cell of the present invention may be HSC (hematopoietic stem cell). For example, the cell of the present invention may be obtained by differentiation of HSC cells. In some embodiments, T cells are type 1 T helper cells and type 2 T helper cells. In some embodiments, the T cells expressing this receptor are αβ-T cells. In alternative embodiments, the T cells expressing this receptor are γδ-T cells.

For example, when a cell expresses the TCR of the invention, the engineered TCR is expressed at a similar or improved level on the cell surface, exhibits similar or higher functional activity (e.g., cell killing activity) and/or similar or higher anti-tumor activity, compared to the expression level, functional activity, and/or anti-tumor activity of the same TCR in similar cells (but in which the expression of the endogenous TCR has been reduced or eliminated). For example, when expressed in a cell, the expression level of the engineered TCR as described herein on the cell surface is at least about 80% to 120%, such as at least about 80%, 85%, 90%, 95%, 100%, 105%, 110%, 115% or 120% of the endogenous TCR expressed by the cell. For example, a cell expressing the TCR of the present invention or its antigen-binding fragment can exhibit NKT cell-related cellular activity.

In some embodiments, the TCR or its antigen binding fragment or the TCR derived binding molecule as described herein can increase the immune response, activity or quantity of cells by at least 10% to 20 times, such as at least 10%, 20%, 30%, 40%, 50%, 60%, 70%, 80%, 90%, 100%, 2 times, 3 times, 4 times, 5 times, 10 times, 15 times or 20 times. In some embodiments, the TCR or its antigen binding fragment or the TCR derived binding molecule can increase the serum concentration of IFN-γ when there is an antigen of interest. In some embodiments, activation can induce an increase in serum concentration of IFN-γ by at least 10% to 1000 times, such as at least 10%, 20%, 30%, 40%, 50%, 60%, 70%, 80%, 90%, 1 times, 2 times, 5 times, 10 times, 50 times, 100 times, 200 times, 300 times, 400 times, 500 times, 600 times, 700 times, 800 times, 900 times or 1000 times. In some embodiments, activation can induce an increase in specific killing of target cells by at least 10% to 5 times, such as at least 10%, 20%, 30%, 40%, 50%, 60%, 70%, 80%, 90%, 1 times, 2 times, 3 times, 4 times or 5 times. In some embodiments, the specific killing of target cells is determined by the killing efficacy of target cells using the methods described herein.

In one aspect, the present invention provides a method for preparing the TCR of the present invention, the method comprising culturing the cells of the present invention under conditions such that the TCR is expressed.

In one aspect, the present invention provides a kit, which comprises the TCR or its antigen-binding fragment described in the present invention, the fusion protein described in the present invention, the nucleic acid molecule described in the present invention, the vector described in the present invention, the composition described in the present invention and/or the cell described in the present invention.

In one aspect, the present invention provides a method for culturing cells, which may comprise administering the TCR or antigen-binding fragment thereof described in the present invention, the fusion protein described in the present invention, the nucleic acid molecule described in the present invention, the vector described in the present invention, the composition described in the present invention, the cell described in the present invention and/or the kit described in the present invention. For example, the method of culturing cells of the present invention can comprise transducing the cells with a vector expressing the TCR or fusion protein of the present invention.

In one aspect, the present invention provides the use of the TCR or antigen-binding fragment thereof, the fusion protein, the nucleic acid molecule, the vector, the composition, the cell and/or the kit in the preparation of a drug, wherein the drug is used to prevent and/or treat a disease and/or symptom. For example, the disease comprises a tumor.

In one aspect, the present invention provides the TCR or antigen-binding fragment thereof of the present invention, the fusion protein of the present invention, the nucleic acid molecule of the present invention, the vector of the present invention, the composition of the present invention, the cell of the present invention and/or the kit of the present invention, which can be used to prevent, alleviate and/or treat a disease. For example, the disease comprises a tumor.

In one aspect, the present invention provides a method for preventing, alleviating and/or treating a disease, wherein the method comprising providing the TCR or antigen-binding fragment thereof described in the present invention, the fusion protein described in the present invention, the nucleic acid molecule described in the present invention, the vector described in the present invention, the composition described in the present invention, the cell described in the present invention and/or the kit described in the present invention. For example, the disease comprises a tumor.

According to the present invention, the tumor is selected from lymphoma, melanoma, ovarian cancer, cervical cancer, lung cancer, bladder cancer, breast cancer, head and neck cancer, pancreatic cancer, liver cancer, gastric cancer, colorectal cancer and kidney cancer.

Not wishing to be bound by any theory, the following examples are merely intended to illustrate the proteins, methods and uses of the present invention, and are not intended to limit the scope of the present invention.

### EXAMPLES

### Example 1. Flow Cytometry and Single Cell Sequencing

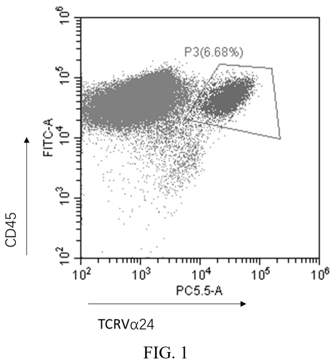

The PBMC cells of donor 1 were cultured in X-VIVO 15 medium, and 500 IU IL-2, 10% FBS, and 100 ng/ml α-GalCer were added to the medium. After 14 days of culture, TCRVα24-positive NKT cells were sorted out using a cell flow cytometer (the antibody used was Biolegend 360004). FIG. 1 shows the results of sorting the CD45-positive TCRVα24-positive cell population.

The 10X Genomic single-cell sequencing sample preparation kit was used to label and amplify the TCR sequences of the sorted cells, and the corresponding transcriptome was extracted and reverse transcribed. The obtained TCR sequences and cDNA library were subsequently sequenced, and the obtained TCR sequences were sorted by abundance as shown in Table 1.

**Table 1**

| ID | TCRA chain | TCRB chain | Freq |
|---|---|---|---|
| TCR-003 | TRAV10_TRAJ18 | TRBV25-1_TRBD1_TRBJ2-1 | 332 |
| TCR-006 | TRAV36/DV7_TRAJ45 | TRBV9- | 85 |
| TCR-007 | TRAV10_TRAJ18 | TRBV25-1_NA_TRBJ1-2 | 60 |
| TCR-001 | TRAV10_TRAJ18 | TRBV25-1_TRBD1_TRBJ2-7 | 57 |
| TCR-not detected | TRAV10; TRAV1-1_TRAJ18; TRAJ39 | TRBV25-1_TRBD2_TRBJ2-3 | 51 |
| TCR-002 | TRAV10_TRAJ18 | TRBV25-1_NA_TRBJ2-1 | 50 |
| TCR-not detected | TRAV4_TRAJ31 | TRBV20-1_NA_TRBJ2-1 | 35 |
| TCR-004 | TRAV10_TRAJ18 | TRBV25-1_TRBD2_TRBJ2-3 | 34 |
| TCR-not detected | TRAV10; TRAV22_TRAJ18; TRAJ52 | TRBV25-1_NA_TRBJ2-1 | 27 |
| TCR-not detected | TRAV10_TRAJ18 | TRBV25-1NA_TRBJ2-1 | 27 |
| TCR-not detected | TRAV25_TRAJ26 | TRBV12-3_NA_TRBJ2-7 | 24 |
| TCR-not detected | TRAV10_TRAJ18 | TRBV25-1_NA_TRBJ2-7 | 23 |
| TCR-not detected | TRAV12-3_TRAJ54 | TRBV6-5_NA_TRBJ1-5 | 21 |
| TCR-not detected | TRAV10; TRAV6_TRAJ18; TRAJ43 | TRBV25-1_TRBD1_TRBJ2-7 | 21 |
| TCR-not detected | TRAV10_TRAJ18 | TRBV25-1_NA_TRBJ2-5 | 20 |
| TCR-not detected | TRAV10_TRAJ18 | TRBV25-1_NA_TRBJ2-1 | 20 |
| TCR-005 | TRAV10_TRAJ18 | TRBV25-1_TRBD1_TRBJ2-7 | 19 |
| TCR-not detected | TRAV10_TRAJ18 | TRBV25-1_NA_TRBJ2-1 | 19 |
| TCR-not detected | TRAV8-3; TRAV13-1; TRAV12-2_TRAJ53; TRAJ48; TRAJ29 | TRBV28_NA_TRBJ2-7 | 16 |
| TCR-not detected | TRAV13-1_TRAJ3 | TRBV7-9_TRBD1_TRBJ2-7 | 13 |

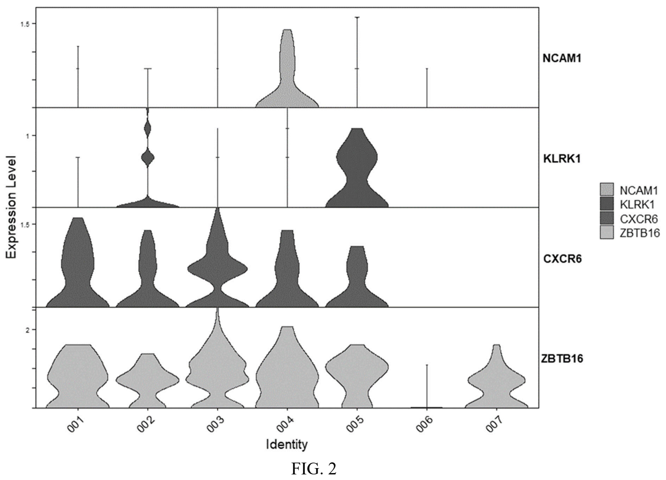

FIG. 2 shows the results of single-cell transcriptome sequencing of cells corresponding to each group of TCR. After analysis and screening, the results show that the cell populations of TCR-001, 002, 003, 004, and 005 corresponding to the TCR of the present invention can centrally express 4 NKT-related marker genes (NCAM1 (CD56), KLRK1 (NKG2D), CXCR6, and ZBTB16). Therefore, the TCR of the present invention can have a high correlation with NKT cells. Other TCR sequences, such as TCR-006 or TCR-007, may have no correlation with NKT cells.

Among them, the cell population corresponding to the V+D+J region of the TCRB chain shown in SEQ ID NO: 1 is TCR-001,

The cell population corresponding to the V+D+J region of the TCRB chain shown in SEQ ID NO: 2 is TCR-002.

The cell population corresponding to the V+D+J region of the TCRB chain shown in SEQ ID NO: 3 is TCR-003.

The cell population corresponding to the V+D+J region of the TCRB chain shown in SEQ ID NO: 4 is TCR-004.

The cell population corresponding to the V+D+J region of the TCRB chain shown in SEQ ID NO: 5 is TCR-005.

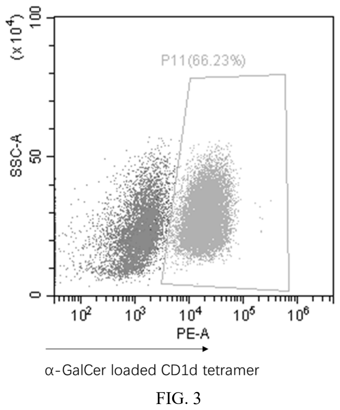

Similarly, the PBMC cells of donor 2 were stimulated with 100 ng/ml α-GalCer and expanded. They were then stained with α-GalCer-loaded CD1d tetramer that recognizes NKT TCR (the tetramer used was MBL TS-HCG-1), and the tetramer-positive cell population was sorted by flow cytometry. FIG. 3 shows the results of the tetramer-positive cell population sorting.

The sequencing steps were the same as in Example 1. The 10X Genomics single-cell sequencing sample preparation kit was used to perform TCR sequence tagging and corresponding cDNA library construction. The obtained TCR sequences were sorted by abundance as shown in Table 2.

**Table 2**

| Group | Freq | TRB_V | TRB_J | TRA_V | TRA_J |
|---|---|---|---|---|---|
| TCR-010 | 667 | TRBV25-1 | TRBJ2-7 | TRAV10 | TRAJ18 |
| TCR-011 | 168 | TRBV25-1 | TRBJ2-1 | TRAV10 | TRAJ18 |
| TCR-012 | 131 | TRBV25-1 | TRBJ1-5 | TRAV10 | TRAJ 18 |
| TCR-009 | 97 | TRBV25-1 | TRBJ2-7 | TRAV10 | TRAJ18 |
| TCR-008 | 96 | TRBV25-1 | TRBJ2-7 | TRAV10 | TRAJ18 |
| TCR-013 | 81 | TRBV25-1 | TRBJ1-4 | TRAV10 | TRAJ18 |
| TCR-014 | 51 | TRBV25-1 | TRBJ2-7 | TRAV10 | TRAJ18 |
| TCR-015 | 27 | TRBV25-1 | TRBJ2-1 | TRAV10 | TRAJ18 |

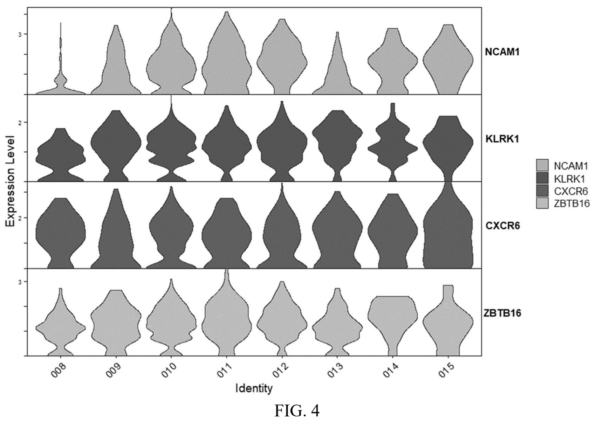

FIG. 4 shows the results of single-cell transcriptome sequencing of cells corresponding to each group of TCR. After analysis and screening, the results show that the cell populations of TCR-008 to 015 corresponding to the TCR of the present invention can centrally express 4 NKT-related marker genes (NCAM1 (CD56), KLRK1 (NKG2D), CXCR6, ZBTB16). Therefore, the TCR of the present invention can be highly correlated with NKT cells.

Among them, the cell population corresponding to the V+D+J region of the TCRB chain shown in SEQ ID NO: 6 is TCR-008,
the cell population corresponding to the V+D+J region of the TCRB chain shown in SEQ ID NO: 7 is TCR-009,
the cell population corresponding to the V+D+J region of the TCRB chain shown in SEQ ID NO: 8 is TCR-010,
the cell population corresponding to the V+D+J region of the TCRB chain shown in SEQ ID NO: 9 is TCR-011,
the cell population corresponding to the V+D+J region of the TCRB chain shown in SEQ ID NO: 10 is TCR-012,
the cell population corresponding to the V+D+J region of the TCRB chain shown in SEQ ID NO: 11 is TCR-013,
the cell population corresponding to the V+D+J region of the TCRB chain shown in SEQ ID NO: 12 is TCR-014;
the cell population corresponding to the V+D+J region of the TCRB chain shown in SEQ ID NO: 13 is TCR-015.

### Example 2. TCR Expression Detection

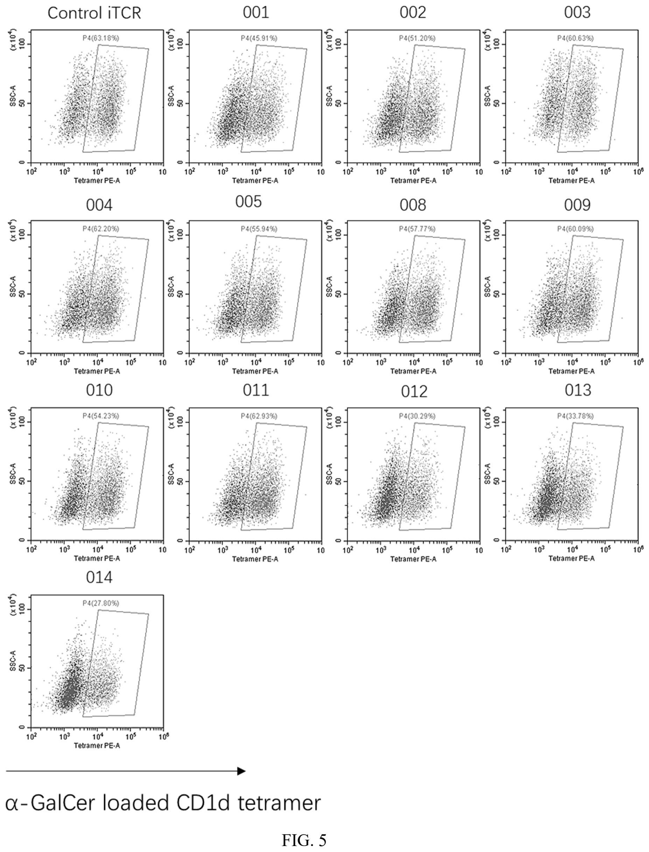

Plasmids expressing the iNKT TCR of the present invention and the disclosed iNKT TCR (control iTCR, whose α chain is shown in SEQ ID NO: 38, and whose β chain is shown in SEQ ID NO: 37) were constructed and packaged with lentivirus, and used to transduce Jurkat cells in which the endogenous TCR had been knocked out. Expression was detected using CD1d tetramer loaded with α-GalCer (FIG. 5). The results showed that the iNKT TCR of the present invention can be expressed in Jurkat and is specifically bound by CD1d tetramer loaded with α-GalCer.

HSC cells were transduced with lentivirus expressing the iNKT TCR of the present invention, and the expression of the cells was detected during the differentiation process. The results showed that the TCR of the present invention can be normally expressed on the cell membrane at the terminal stage of differentiation and was conducive to the differentiation of HSC cells into T cells.

### Example 3. TCR-expressing cell function experiment

### Detection of signal strength of the transduced TCR in Jurkat cells by luciferase activity downstream of TCR

Jurkat cell lines with NFAT-Luc reporter were purchased from BPS Bioscience, and the TCR α chain and β chain were knocked out. Control iTCR and iNKT TCR of the present invention were transferred by lentivirus, respectively. After confirming the expression with α-GalCer-CD1d tetramer, they were mixed and cultured with C1R cells expressing CDld molecules (C1R-CD1d cell line) at a ratio of 1:1 in the presence or absence of 100 ng/ml α-GalCer in culture medium overnight. The next day, the cells were detected using the One-Lite Luciferase assay system (Vazyme) kit, and the microplate reader used for the detection was BioTek Synergy HTX (Table 3). The results showed that many of the iNKT TCRs in the present invention showed responses to α-GalCer-CD1d stimulation that were similar to or even better than those of the control iTCR (reflected by the ratio of +α-GalCer/-α-GalCer, where +α-GalCer indicated GalCer stimulation and -α-GalCer indicated no GalCer stimulation). This experiment was performed twice, and the results of the two experiments were taken. The average of the ratios of +α-GalCer/-α-GalCer was taken as the average responding fold.

**Table 3**

| Group | Average responding fold | Group | Average responding fold |
|---|---|---|---|
| iTCR ctrl | B | TCR-009 | B |
| TCR-001 | B | TCR-010 | C |
| TCR-002 | A | TCR-011 | B |
| TCR-003 | B | TCR-012 | A |
| TCR-004 | B | TCR-013 | A |
| TCR-005 | C | TCR-014 | C |
| TCR-008 | B | | |

Among them, the average responding fold was classified into A, B, and C:
A: greater than 1 time, less than or equal to 3 times;
B: greater than 3 times, less than or equal to 5 times;
C: more than 5 times,

### Cell expansion

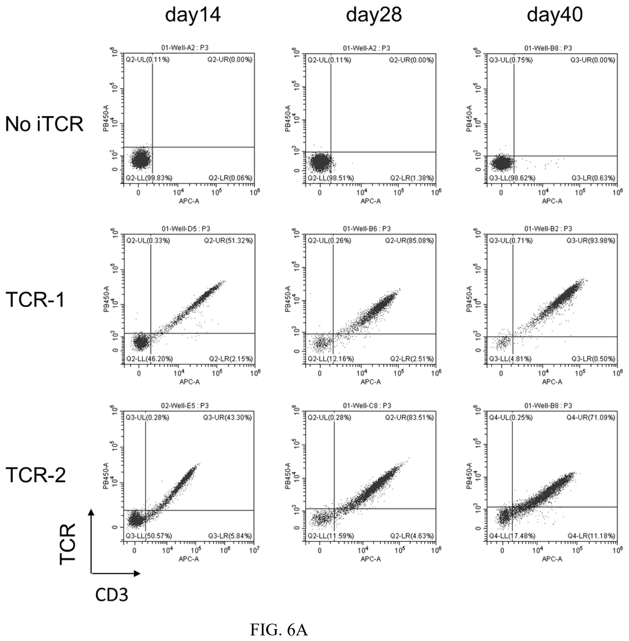

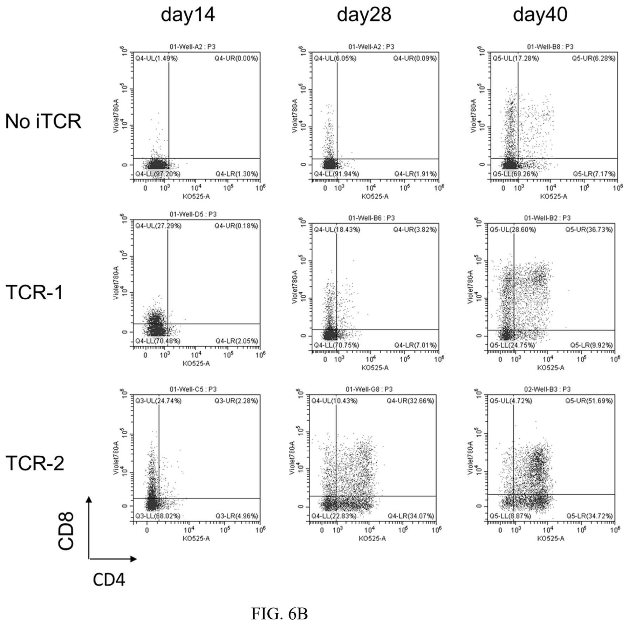

iNKT cells expressing the iNKT TCR of the present invention were harvested from HSC differentiation. FIGs. 6A-6B show that during the differentiation process, the T cell phenotypes such as CD3 and CD4/CD8 of the cells expressing the iTCR of the present invention were significantly better than those of the group without iTCR (no iTCR).

At the same time, the fold expansion at different stages of differentiation in the iTCR group were significantly better than those in the group without iTCR (Table 4);

**Table 4**

| Group | Phase 1 from 0 to 14 days | Phase 2 from 14 to 28 days | Phase 3 from 28 to 40 days |
|---|---|---|---|
| | Fold expansion | Fold expansion | Fold expansion |
| No iTCR | 66 | 7.6 | 1 |
| TCR-1 | 885 | 48 | 6 |
| TCR-2 | 1030.5 | 51 | 4 |

During the PBMC trophoblast expansion stage, the effect of PBMC trophoblasts in promoting the expansion of iNKT cells derived from HSC differentiation expressing iNKT TCR was significantly better than that of the group without iTCR (Table 5).

**Table 5**

| Group | Fold expansion of CB-iNKT cells (PBMC feeder cell activation) |
|---|---|
| No iTCR | 5.1 |
| TCR-1 | 10.69 |
| TCR-2 | 27.76 |

### Cell killing

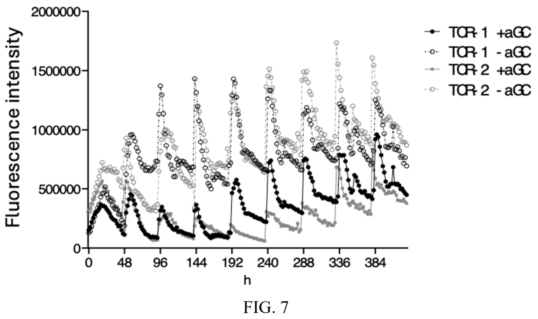

The iNKT cells differentiated from HSCs and expanded by trophoblast stimulation were harvested and co-cultured with the target cell C1R-CD1d-mcherry cell line at an effector-target ratio of E:T=1:4. 100 ng/ml α-GalCer was added to the culture medium. After co-culture, the residual target cells were detected by Incucyte. Target cells were added every 48 hours to complete 9 rounds of in vitro killing. The results showed that the iTCR of the present invention can activate HSC-differentiated cells and effectively kill target cells (FIG. 7).

### Cell killing

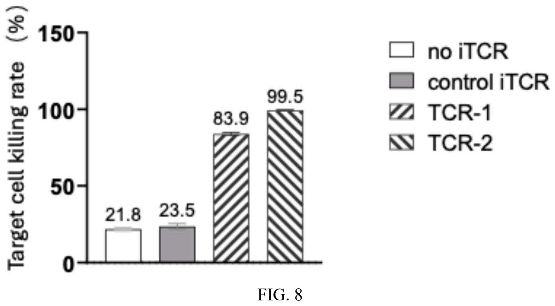

T cells derived from peripheral blood (PBMC) were harvested to express the iTCR of the present invention, as well as a disclosed iTCR (control iTCR, whose α chain is shown in SEQ ID NO: 38, and whose β chain is shown in SEQ ID NO: 37), and co-cultured with the target cell C1R-CD1d-mcherry cell line at an effector-target ratio of E:T=1:4. 100 ng/ml α-GalCer was added to the culture medium. After 96 hours, the residual target cells were detected, and the killing rate of the effector cells against the target cells was calculated (FIG. 8). The results showed that the cells expressing the iTCR of the present invention can effectively kill the target cells, and the killing effect is better than that of the known iTCR.

The foregoing detailed description is provided by way of explanation and examples and is not intended to limit the scope of the appended claims. Various changes to the embodiments of the present invention are obvious to those of ordinary skill in the art and are intended to be within the scope of the appended claims and their equivalents.

## Claims

1. A T cell receptor (TCR) or an antigen binding fragment thereof, wherein the TCR comprises a β chain variable region (Vβ), the Vβ comprises a CDR3, and the CDR3 of the Vβ comprises the sequence shown in any one of SEQ ID NOs: 14-26.

2. The TCR or antigen-binding fragment thereof of claim 1, wherein the TCR comprises a β chain variable region (Vβ), the Vβ comprises CDR1 and CDR2, the CDR1 of the Vβ comprises the sequence shown in SEQ ID NO: 28, and the CDR2 of the Vβ comprises the sequence shown in SEQ ID NO: 27.

3. The TCR or antigen-binding fragment thereof according to any one of claims 1-2, wherein the TCR comprises a β chain variable region (Vβ), and the Vβ comprises the sequence shown in any one of SEQ ID NOs: 1-13.

4. The TCR or antigen-binding fragment thereof according to any one of claims 1-3, wherein the TCR comprises a β chain constant region, and the β chain constant region comprises the sequence shown in SEQ ID NO: 29 or 30.

5. The TCR or antigen-binding fragment thereof according to any one of claims 1-4, wherein the TCR comprises an α chain variable region (Vα), the Vα comprises CDR3, and the CDR3 of the Vα comprises the sequence shown in SEQ ID NO: 31.

6. The TCR or antigen-binding fragment thereof according to any one of claims 1-5, wherein the TCR comprises an α chain variable region (Vα), the Vα comprises CDR1 and CDR2, the CDR1 of the Vα comprises the sequence shown in SEQ ID NO: 33, and the CDR2 of the Vα comprises the sequence shown in SEQ ID NO: 32.

7. The TCR or antigen-binding fragment thereof according to any one of claims 1-6, wherein the TCR comprises an α chain variable region (Vα), and the Vα comprises the sequence shown in SEQ ID NO: 34.

8. The TCR or antigen-binding fragment thereof according to any one of claims 1-7, wherein the TCR comprises an α chain constant region, and the α chain constant region comprises the sequence shown in SEQ ID NO: 35.

9. A fusion protein, wherein the fusion protein comprises the TCR or antigen-binding fragment thereof according to any one of claims 1-8.

10. A nucleic acid molecule, wherein the nucleic acid molecule encodes the TCR or antigen-binding fragment thereof according to any one of claims 1-8 and/or the fusion protein according to claim 9.

11. A vector, wherein the vector comprises the nucleic acid molecule of claim 10.

12. A composition comprising the TCR or antigen-binding fragment thereof according to any one of claims 1-8, the fusion protein according to claim 9, the nucleic acid molecule according to claim 10, and/or the vector according to claim 11, and optionally a pharmaceutically acceptable adjuvant.

13. A cell, wherein the cell comprises the TCR or antigen-binding fragment thereof according to any one of claims 1-8, the fusion protein according to claim 9, the nucleic acid molecule according to claim 10, the vector according to claim 11 and/or the composition according to claim 12.

14. The cell according to claim 13, wherein the cell comprises an immune cell.

15. The cell according to any one of claims 13 to 14, wherein the cell comprises a NK cell, a NKT cell and/or a T cell.

16. The cell according to any one of claims 13-15, wherein the cell comprises a cell derived from an artificial pluripotent stem cell (iPSC), a PBMC cell and/or a tumor infiltrating lymphocyte cell.

17. A kit, wherein the kit comprises the TCR or antigen-binding fragment thereof according to any one of claims 1-8, the fusion protein according to claim 9, the nucleic acid molecule according to claim 10, the vector according to claim 11, the composition according to claim 12 and/or the cell according to any one of claims 13-16.

18. A method for influencing cell growth, comprising administering the TCR or antigen-binding fragment thereof according to any one of claims 1-8, the fusion protein according to claim 9, the nucleic acid molecule according to claim 10, the vector according to claim 11, the composition according to claim 12, the cell according to any one of claims 13-16 and/or the kit according to claim 17.

19. Use of the TCR or antigen-binding fragment thereof according to any one of claims 1-8, the fusion protein according to claim 9, the nucleic acid molecule according to claim 10, the vector according to claim 11, the composition according to claim 12, the cell according to any one of claims 13-16 and/or the kit according to claim 17 in the preparation of a medicament, wherein the medicament is used to prevent and/or treat a disease and/or symptom.

20. A drug for preventing and/or treating a disease and/or symptom, comprising the TCR or antigen-binding fragment thereof according to any one of claims 1-8, the fusion protein according to claim 9, the nucleic acid molecule according to claim 10, the vector according to claim 11, the composition according to claim 12, the cell according to any one of claims 13-16 and/or the kit according to claim 17 as an active ingredient.

21. A method for preventing and/or treating a disease and/or symptom, comprising administering to a subject in need thereof the TCR or antigen-binding fragment thereof according to any one of claims 1-8, the fusion protein according to claim 9, the nucleic acid molecule according to claim 10, the vector according to claim 11, the composition according to claim 12, the cell according to any one of claims 13-16 and/or the kit according to claim 17.

22. The TCR or antigen-binding fragment thereof according to any one of claims 1-8, the fusion protein according to claim 9, the nucleic acid molecule according to claim 10, the vector according to claim 11, the composition according to claim 12, the cell according to any one of claims 13-16 and/or the kit according to claim 17, which is used for preventing and/or treating a disease and/or symptom.

23. The use according to claim 19, the medicament according to claim 20, the method according to claim 21, and/or the TCR or its antigen-binding fragment, fusion protein, nucleic acid molecule, vector, composition, cell and/or kit for use according to claim 22, wherein the disease and/or symptom comprises a tumor.

24. The use according to claim 19, the medicament according to claim 20, the method according to claim 21, and/or the TCR or its antigen-binding fragment, fusion protein, nucleic acid molecule, vector, composition, cell and/or kit for use according to claim 22, wherein the disease and/or symptom comprises a hematological tumor and/or a solid tumor.

25. The use according to claim 19, the medicament according to claim 20, the method according to claim 21, and/or the TCR or its antigen-binding fragment, fusion protein, nucleic acid molecule, vector, composition, cell and/or kit for use according to claim 22, wherein the disease and/or symptom comprises one or more selected from the following group: lymphoma, melanoma, ovarian cancer, cervical cancer, lung cancer, bladder cancer, breast cancer, head and neck cancer, pancreatic cancer, liver cancer, gastric cancer, colorectal cancer and kidney cancer.
